# EUROPÄISCHE PATENTSCHRIFT

(11) **EP 1 334 081 B1**
(45) Veröffentlichungstag und Bekanntmachung des Hinweises auf die Patenterteilung: **04.03.2009**
(21) Anmeldenummer: 01993597.2
(22) Anmeldetag: 13.11.2001
(51) Int. Cl.: C07C 69/80, C07C 69/44, C07C 67/08, C07C 67/54, C08G 63/78

(54) **VERFAHREN ZUR HERSTELLUNG VON ESTERN MEHRBASIGER SÄUREN**
METHOD FOR PRODUCING ESTERS OF MULTIBASIC ACIDS
PROCEDE DE PREPARATION D'ESTERS D'ACIDES POLYBASIQUES

(30) Priorität: 13.11.2000 DE 10056179
(43) Veröffentlichungstag der Anmeldung: 13.08.2003
(73) Patentinhaber: BASF SE, 67056 Ludwigshafen (DE)
(72) Erfinder: DISTELDORF, Walter, 67157 Wachenheim (DE); GOLFIER, Günther, 67227 Frankenthal (DE); MORSBACH, Bernd, 67069 Ludwigshafen (DE); SCHWIRTEN, Kurt, 67227 Frankenthal (DE)
(74) Vertreter: Pohl, Michael Friedrich
(86) Internationale Anmeldenummer: PCT/EP2001/013131
(87) Internationale Veröffentlichungsnummer: WO 2002/038531

(56) Entgegenhaltungen:
- EP-A- 0 721 928
- DE-A- 1 618 395
- DE-B- 1 185 611
- US-A- 5 324 853
- P. M. LORZ ET AL.: "ULLMANN'S ENCYCLOPEDIA OF INDUSTRIAL CHEMISTRY. ELECTRONIC RELEASE 1999" 1999 , WILEY-VCH , WEINHEIM, DEUTSCHLAND XP002193336 PHTHALIC ACID AND DERIVATIVES - PHTHALATES 5.3 PRODUCTION

## Beschreibung

Die vorliegende Erfindung betrifft ein Verfahren zur Herstellung von Estern einer unter mehrbasigen C₄-C₁₀-Carbonsäuren ausgewählten Säurekomponente und einer unter C₃-C₁₂-Alkanolen oder -Alkandiolen ausgewählten Alkoholkomponente, deren Alkylkette durch 1 bis 3 Sauerstoffatome unterbrochen sein kann.

Ester der genannten Art werden in grossem Umfang als Weichmacher in Kunststoffen wie Celluloseacetaten, Polyurethanen, PVC, Polyacrylaten usw. verwendet. Sie sind durch Umsetzung der Säurekomponente oder eines Anhydrids davon mit der Alkoholkomponente in Gegenwart eines Veresterungskatalysators herstellbar. Die Umsetzung verläuft in einer Gleichgewichtsreaktion. Das Gleichgewicht kann auf die Seite der Produkte, d. h. des Esters, verschoben werden, indem das als Nebenprodukt entstehende Reaktionswasser kontinuierlich entfernt wird. Wenn der verwendete Alkohol eine Mischungslücke mit Wasser aufweist, kann man aus dem Reaktionsgemisch kontinuierlich ein Gemisch von Reaktionswasser und Alkohol abdestillieren, und nach der Phasentrennung die organische Phase zurück in die Veresterung führen, während die wässrige Phase aus dem System entfernt wird. Da sich Wasser stets in mehr oder weniger grossen Mengen im Alkohol löst, wird mit der organischen Phase auch Wasser zurückgeführt, so dass die Verschiebung des Gleichgewichts zu einem quantitativen Umsatz mitunter sehr langsam verläuft.

Bei Verwendung von Ethylenglykolmonobutylether existiert z. B. nur zwischen 43°C und 133°C eine Mischungslücke mit Wasser (vgl. G. Schneider und G. Wilke, Zeitschr. f. Phys. Chemie, NF, 20, 219 (1959), so daß in diesem Fall der Phasenscheider nachteiligerweise auf einer Temperatur zwischen den beiden Entmischungspunkten gehalten werden muss. Ausserdem enthält die zurücklaufende Alkoholphase noch 39,7 Gew.-% Wasser.

DE-A-1185611 beschreibt die Veresterung von Carbonsäuren mit Alkoholen in Gegenwart eines Titanperoxidkatalysators. Das bei der Veresterungsreaktion gebildete Wasser wird abdestilliert und in einem Phasenabscheider von mitdestilliertem Alkohol getrennt, der dann in die Reaktion zurückgeführt wird.

US 5,324,853 beschreibt die Veresterung von Säuren oder Anhydriden mit Alkoholen unter destillativer Entfernung des gebildeten Reaktionswassers. Eine Abtrennung und Rückführung des mit dem Reaktionswasser abdestillierten Alkohols wird nicht beschrieben.

Der vorliegenden Erfindung liegt die Aufgabe zu Grunde, ein Verfahren zur Herstellung der eingangs genannten Ester bereitzustellen, das einfach durchzuführen ist und in kurzer Zeit zu einem im Wesentlichen quantitativen Umsatz führt.

Erfindungsgemäss wird diese Aufgabe durch ein Verfahren gelöst, bei dem man
a) in einer Reaktionszone ein im wesentlichen aus der Säurekomponente oder einem Anhydrid davon und der Alkoholkomponente bestehendes Gemisch in Gegenwart eines Veresterungskatalysators zum Sieden erhitzt,
b) die Alkohol und Wasser enthaltenden Dämpfe rektifikativ in eine alkoholreiche Fraktion und eine wasserreiche Fraktion auftrennt,
c) die alkoholreiche Fraktion in die Reaktionszone zurückführt und die wasserreiche Fraktion aus dem Verfahren ausleitet.

Bei der Säurekomponente handelt es sich um eine mehrbasige, insbesondere zweibasige, Carbonsäure mit 4 bis 10 Kohlenstoffatomen. Sie kann aromatisch oder aliphatisch sein. In Betracht kommen beispielsweise Bernsteinsäure, Glutarsäure, Adipinsäure, Pimelinsäure, Korksäure, Azelainsäure, Sebacinsäure, Hexahydrophthalsäure, Hexahydroisophthalsäure oder Hexahydroterephthalsäure, sowie Phthalsäure, Isophthalsäure oder Terephthalsäure. Davon sind Adipinsäure und Phthalsäure besonders bevorzugt. Die Säurekomponente kann als solche oder in Form eines Anhydrids, z. B. eines intra- oder intermolekularen Anhydrids, eingesetzt werden. So wird Phthalsäure üblicherweise in Form des technisch verfügbaren Phthalsäureanhydrids eingesetzt.

Die Alkoholkomponente ist ausgewählt unter Hexanol, 2-Ethylhexanol und Ethylenglykolmonobutylether.

Bezogen auf die Säurekomponente wird die Alkoholkomponente vorzugsweise im molaren Überschuss eingesetzt, z. B. in einem Überschuss von bis zu 500 %, vorzugsweise bis zu 200 %, insbesondere bis zu 100 %, besonders bevorzugt von 20 bis 60 %, über die stöchiometrisch erforderliche Menge. Das erfindungsgemässe Verfahren wird in Abwesenheit eines externen Schleppmittels durchgeführt.

Als Veresterungskatalysatoren kommen übliche, gewöhnlicherweise für Veresterungsreaktionen eingesetzte Katalysatoren in Betracht. Hierzu zählen Mineralsäuren, wie Schwefelsäure, Phosphorsäure; organische Sulfonsäuren, wie Methansulfonsäure, p-Toluolsulfonsäure; Lewis-Säuren, insbesondere Titan-, Zinn(IV)- oder Zirkoniumverbindungen, wie Tetraalkoxytitane, z. B. Tetrabutoxytitan, und Z inn(IV)-oxid.

Der Veresterungskatalysator wird in einer wirksamen Menge eingesetzt, die üblicherweise im Bereich von 0,05 bis 5 Gew.-%, vorzugsweise 0,1 bis 1 Gew.-%, bezogen auf die Summe von Säurekomponente (oder Anhydrid) und Alkoholkomponente, liegt.

Das Gemisch von Säurekomponente oder dem Anhydrid davon, Alkoholkomponente und Veresterungskatalysator wird zum Sieden erhitzt. Die hierzu erforderlichen Bedingungen von Druck und Temperatur hängen stark von der verwendeten Alkoholkomponente ab. Als allgemeiner Rahmen lassen sich eine Temperatur von 140 bis 220°C und ein Druck von 0,3 bis 2 bar angeben. Vielfach ist es vorteilhaft, im Verlauf der Reaktion die Temperatur anzuheben und/oder den Druck abzusenken.

Zur Durchführung des erfindungsgemässen Verfahrens sind alle gängigen beheizbaren Reaktoren geeignet, wie z. B. ein Rührkesselreaktor.

Zweckmässigerweise leitet man die Alkohol und Wasser enthaltenden Dämpfe zur rektifikativen Trennung in eine Kolonne und führt das vom unteren Ende der Kolonne ablaufende Produkt, d. h. das Sumpfprodukt, als alkoholreiche Fraktion in die Reaktionszone zurück und leitet das Destillat aus dem Verfahren aus.

Bei der Kolonne kann es sich um eine übliche, dem Fachmann bekannte Kolonne, wie eine Füllkörperkolonne oder eine Bodenkolonne handeln. Bei den Füllkörpern kann es sich um solche aus Glas, keramischem Material oder Metall handeln, z. B. Raschig-Ringe, Sattelkörper, Kugeln, Spiralen, Ringe und dergleichen. Die Kolonne weist geeigneterweise z. B. 8 bis 12 theoretische Böden auf. Das untere Ende der Kolonne steht in gasdurchlässiger Verbindung mit dem Dampfraum der Reaktionszone. Der am oberen Ende der Kolonne austretende rektifizierte Dampf wird zweckmässigerweise vollständig kondensiert. Das Kondensat wird ganz oder teilweise als Destillat abgenommen; der nicht als Destillat abgenommene Teil wird als Rücklauf in die Kolonne zurückgeführt. Ein Rücklaufverhältnis R/D von 1:2 bis 1:10 hat sich als besonders zweckmässig erwiesen. Ist das Kondensat zweiphasig, ist es zweckmäßig, die organische Phase vollständig zurückzuführen und die wässrige Phase zumindest teilweise abzuziehen.

Die Begriffe "alkoholreiche" Fraktion und "wasserreiche" Fraktion" sollen bedeuten, dass die jeweilige Fraktion gegenüber der Zusammensetzung der aus der Reaktionszone abgezogenen Dämpfe Alkohl-angereichert bzw. Wasser-angereichert ist. Die alkoholreiche Fraktion weist vorzugsweise einen Alkoholgehalt von mehr als 80 Gew.-%, insbesondere mehr als 95 Gew.-%, auf; besonders bevorzugt handelt es sich dabei um im wesentlichen reinen Alkohol.

Nach Erreichen des gewünschten Umsatzes bricht man bei diskontinuierlicher Betriebsweise die Reaktion im Allgemeinen ab und destilliert die überschüssige Alkoholkomponente ab. Aus dem Rückstand kann nach üblichen Verfahren, z. B. durch Extraktion zur Entfernung des Katalysators oder der Hydrolyseprodukte davon und anschliessende Destillation, der gewünschte Ester isoliert werden. Das erfindungsgemässe Verfahren kann alternativ kontinuierlich durchgeführt werden, wobei man hierzu zweckmässigerweise eine Anordnung von Haupt- und Nachreaktionszone oder eine Kaskade von Reaktionszonen anwendet. Hierbei werden die Dämpfe wenigstens einer der Reaktionszonen, vorzugsweise der Hauptreaktionszone, wie beschrieben aufgetrennt, wobei die Dämpfe der einzelnen Reaktoren für sich rektifiziert oder vereinigt und gemeinsam rektifiziert werden können. Der Rücklauf der alkoholreichen Fraktion erfolgt in die jeweilige Reaktionszone, oder sie wird nach Bedarf auf die einzelnen Reaktionszonen aufgeteilt.

Die wasserreiche Fraktion wird zweckmässigerweise zur Rückgewinnung der darin enthaltenen Alkoholkomponente aufgearbeitet. Vielfach kann man sich - wie im Falle des Ethylenglykolmonobutylethers - den Umstand zunutze machen, dass die Zusammensetzung der wasserreichen Fraktion innerhalb der Mischungslücke der Alkoholkomponente mit Wasser liegt. Für den Fall des Ethylenglykolmonobutylethers tritt die Mischungslücke nur bei Temperaturen zwischen 43°C und 133°C auf. Daher bringt man die wasserreiche Fraktion hier geeigneterweise auf eine Temperatur im genannten Bereich, vorzugsweise etwa 60-90°C,insbesondere 70-80°C, wobei diese dann in eine organische Phase mit einem Ethylenglykolmonobutylethergehalt von etwa 60,3 Gew.-% und eine wässrige Phase mit etwa 90,4 Gew.-% Wasser zerfällt. Die organische und/oder wässrige Phase können gewünschtenfalls nach üblichen Verfahren weiter aufgereinigt werden.

Die Erfindung wird nun durch die folgenden Beispiele näher veranschaulicht.

### Beispiel 1:

In einem 21-Dreihalskolben mit aufgesetzter Füllkörperkolonne einer Länge von 60 cm mit Kolonnenkopf und Kondensator sowie einem Rücklauf teiler wurden 444g (3 mol) Phthalsäureanhydrid und 1063,8 g (9 mol) Ethylenglykolmonobutylether mit Tetrabutoxytitan (0,8 Gew.-%, bezogen auf den Ansatz) zum Sieden erhitzt, bis im Rückflussteiler deutlicher Rückfluss zu bemerken war. Dann wurde ein Teil des Kondensats der rektifizierten Dämpfe mit einer Temperatur von 99° C entnommen. Nach 120 Minuten waren insgesamt 75 ml Kondensat abgenommen, und der Kolbeninhalt wies bei der Bestimmung des Säuregehaltes eine Säurezahl von 0,056 mg KOH/g auf.

### Vergleichsbeispiel 1

Das Beispiel 1 wurde wiederholt, wobei jedoch anstelle der Füllkörperkolonne auf den Kolben ein Wasserauskreiser aufgesetzt war, dessen Phasenscheider als Doppelmantelgefäss ausgeführt war. Dieses wurde mit einem Thermostaten auf 88° C gehalten. Die obere Phase lief kontinuierlich in den Kolben zurück. Nach 300 Minuten hatte der Kolbeninhalt eine Säurezahl von 9,6, nach 540 Minuten von 0;12.

### Beispiel 2

In einen 2 1 Dreihalskolben mit Rührer und aufgesetzter 50 cm-Kolonne und Kolonnenkopf wurden 451 g (3,1 Mol) Adipinsäure und 792 g (7,75 Mol) n-Hexanol-1 vorgelegt. Man fügte als Katalysator 0,5 % Methansulfonsäure hinzu und erwärmte zum Sieden. Das Kondensat zerfiel in eine organische und eine wasserhaltige Phase. Der Kolonnenkopf wurde so betrieben, daß die organische Phase vollständig zurücklief, während die wässrige Phase sowohl Rücklauf als auch Kopfabzug war. Die Kopftemperatur der Kolonne lag zwischen 95°C und 100°C. Nach 110 Minuten hatte der Kolbeninhalt eine Säurezahl von 0,35.

### Vergleichsbeispiel 2

Das Beispiel 2 wurde wiederholt, wobei die Kolonne durch einen Wasserauskreiser ersetzt wurde. Die organische Phase lief vollständig zurück, während die wässrige Phase abgezogen wurde. Nach 240 Minuten betrug die Säurezahl im Kolbeninhalt 0,7.

## Patentansprüche

1. Verfahren zur Herstellung von Estern einer unter mehrbasigen C₄-C₁₀-Carbonsäuren ausgewählten Säurekomponente und einer unter Hexanol, 2-Ethylhexanol und Ethylenglykolmonobutylether ausgewählten Alkoholkomponente, bei dem man
a) in einer Reaktionszone ein im wesentlichen aus der Säurekomponente oder einem Anhydrid davon und der Alkoholkomponente bestehendes Gemisch in Gegenwart eines Veresterungskatalysators zum Sieden erhitzt,
b) die Alkohol und Wasser enthaltenden Dämpfe rektifikativ in eine alkoholreiche Fraktion mit einem Alkoholgehalt von mehr als 80 Gew.-% und eine gegenüber der Zusammensetzung der aus der Reaktionszone abgezogenen Dämpfe wasserangereicherten Fraktion auftrennt,
c) die alkoholreiche Fraktion in die Reaktionszone zurückführt und die wasserreiche Fraktion aus dem Verfahren ausleitet.

2. Verfahren nach Anspruch 1, bei dem man die Alkohol und Wasser enthaltenden Dämpfe zur Trennung in eine Kolonne leitet und das am unteren Ende der Kolonne ablaufende Produkt als alkoholreiche Fraktion in die Reaktionszone zurückführt und das Destillat als wasserreiche Fraktion aus dem Verfahren ausleitet.

3. Verfahren nach Anspruch 1 oder 2, bei dem die Säurekomponente unter Phthalsäure und Adipinsäure ausgewählt ist.

4. Verfahren nach einem der vorhergehenden Ansprüche, bei dem der Veresterungskatalysator unter Mineralsäuren, organischen Sulfonsäuren und Lewis-Säuren ausgewählt ist.

5. Verfahren nach Anspruch 1 oder 2, bei dem es sich bei der Alkoholkomponente um Ethylenglykolmonobutylether und bei dem Anhydrid der Säurekomponente um Phthalsäureanhydrid handelt.

## Claims

1. A process for preparing esters of an acid component selected from among polybasic C₄-C₁₀ carboxylic acids and an alcohol component selected from among hexanol, 2-ethylhexanol, and ethylene glycol monobutyl ether, by
a) heating at boiling point, in a reaction zone and in the presence of an esterification catalyst, a mixture essentially consisting of the acid component or of an anhydride thereof and of the alcohol component,
b) using rectification to separate the vapor comprising alcohol and water into an alcohol-rich fraction having an alcohol content of more than 80% by weight and a fraction having higher water concentration than the composition of the vapor drawn off from the reaction zone,
c) returning the alcohol-rich fraction into the reaction zone and conducting the water-rich fraction out of the process.

2. The process according to claim 1, in which the vapor comprising alcohol and water is passed into a column for separation, and the product being discharged at the lower end of the column is returned into the reaction zone as alcohol-rich fraction, and the distillate is conducted out of the process as water-rich fraction.

3. The process according to claim 1 or 2, in which the acid component has been selected from among phthalic acid and adipic acid.

4. The process according to any of the preceding claims, in which the transesterification catalyst has been selected from among mineral acids, organic sulfonic acids and Lewis acids.

5. The process according to claim 1 or 2, in which the alcohol component is ethylene glycol monobutyl ether and the anhydride of the acid component is phthalic anhydride.

## Revendications

1. Procédé pour la préparation d'esters d'un composant acide choisi parmi les acides C₄-C₁₀-carboxyliques polybasiques et d'un composant alcool choisi parmi l'hexanol, le 2-éthylhexanol et l'éthylèneglycolmonobutyléther, dans lequel
a) on chauffe à ébullition, dans une zone de réaction, un mélange essentiellement constitué par le composant acide et un anhydride de celui-ci et par le composant alcool, en présence d'un catalyseur d'estérification,
b) on sépare les vapeurs contenant l'alcool et de l'eau par rectification en une fraction riche en alcool, présentant une teneur en alcool supérieure à 80% en poids et en une fraction enrichie en eau par rapport à la composition des vapeurs soutirées de la zone de réaction,
c) on recycle la fraction riche en alcool dans la zone de réaction et on évacue la fraction riche en eau du procédé.

2. Procédé selon la revendication 1, dans lequel on fait passer les vapeurs contenant l'alcool et de l'eau, en vue de la séparation, dans une colonne et on recycle le produit sortant de l'extrémité inférieure de la colonne en tant que fraction riche en alcool dans la zone de réaction et on évacue le distillat en tant que fraction riche en eau du procédé.

3. Procédé selon la revendication 1 ou 2, dans lequel le composant acide est choisi parmi l'acide phtalique et l'acide adipique.

4. Procédé selon l'une quelconque des revendications précédentes, dans lequel le catalyseur d'estérification est choisi parmi les acides minéraux, les acides sulfoniques organiques et les acides de Lewis.

5. Procédé selon la revendication 1 ou 2, dans lequel il s'agit, pour le composant alcool, d'éthylèneglycolmonobutyléther et, pour l'anhydride du composant acide, d'anhydride de l'acide phtalique.
